Europäisches Patentamt

European Patent Office

Office européen des brevets

(19)

(11) **EP 1 188 157 B1**

(12) **EUROPEAN PATENT SPECIFICATION**

(45) Date of publication and mention
of the grant of the patent:
**07.12.2005 Bulletin 2005/49**

(51) Int Cl.⁷: **G08B 13/22**, A61F 5/48,
A61F 13/42, G01F 23/26

(21) Application number: **00938581.6**

(22) Date of filing: **21.06.2000**

(86) International application number:
**PCT/DK2000/000330**

(87) International publication number:
**WO 2000/079497 (28.12.2000 Gazette 2000/52)**

(54) **RADIOFREQUENCY RESONANT CIRCUIT SENSING DEVICE, METHOD OF ITS PRODUCTION, AND USES**

RESONANZDETEKTORSCHALTUNG, PRODUKTIONSWEISE UND GEBRAUCH

DISPOSITIF DE DETECTION PAR CIRCUIT RESONNANT HF, PROCEDE DE PRODUCTION ET UTILISATIONS

(84) Designated Contracting States:
**AT BE CH CY DE DK ES FI FR GB GR IE IT LI LU MC NL PT SE**
Designated Extension States:
**AL LT LV MK RO SI**

(30) Priority: **21.06.1999  DK 88299**

(43) Date of publication of application:
**20.03.2002  Bulletin 2002/12**

(73) Proprietor: **Bent Thorning Bensen A/S**
**3000 Elsinore (DK)**

(72) Inventor: **BENSEN, Bent, Thorning**
**DK-3000 Elsinore (DK)**

(74) Representative: **Wetke, Ellen et al**
**Zacco Denmark A/S**
**Hans Bekkevolds Allé 7**
**2900 Hellerup (DK)**

(56) References cited:
**EP-A2- 0 149 240**     **WO-A1-99/33037**
**US-A- 3 913 219**     **US-A- 4 578 654**
**US-A- 4 843 404**     **US-A- 5 645 932**

**Description**

1. BACKGROUND OF THE INVENTION

The present invention relates to a radiofrequency resonant circuit sensing device, a method of its production, and use thereof for a fluid sensing device for detecting a fluid in an article or a container; for containing a fluid, in particular a human or animal body fluid; and use thereof for radiofrequency resonant circuit tags in detection of theft of articles for sale.

The Technical Field

Generally, radiofrequency resonant circuit devices comprise layered structures of electrically conducting means comprising inductive and capacitive elements, and electrically insulating means separating the electrically conductive layers which are electrically connected through separate perforations of the electrically insulating layers.

Prior art radiofrequency resonant circuit devices include conductive paths that are etched out of various strong metal foils which are laminated on both sides of a flexible insulating carrier foil. Conductive connections are established by through-contacts through the foil which is a rather time consuming process. Further, through-contacts cannot be imprinted because this would damage the layers.

Through-contacts may be avoided by using conductive paths divided into at least two parts electrically connected and applied to one side of the insulating carrier foil so that the two parts of the conductive paths can be brought to overlap by folding with carrier foil therebetween, thereby providing a capacitive element.

One-sided conductive paths on carrier foils can be mass-produced by methods known in the art thereby facilitating cheap production of single-use radiofrequency resonant circuit devices. However, the variability of degree of overlap in the folding process introduces variability in the values of especially the capacitive element of the resonant circuit, thereby deceasing the reliability of the device in some applications.

Variability of the capacitive element may be important in applications of radiofrequency resonant circuit devices for sensing external parameters affecting the function of the resonant circuit.

One such application is detection or deactivation of the resonant circuit by the presence of a fluid such as humidity or a body fluid from a human or an animal.

Generally detection of a fluid, e.g. for detecting the level of a fluid in a container or for detecting a leak of fluid from a body such as a container or a human or animal body discharging said fluid comprises production of a sensor signal of the fluid and detection of the produced sensor signal by appropriate detection electronics.

In applications of e.g. detecting levels or leaks of stationary oil containers, or detecting leak of body fluids from patients or elderly confined to a bed, the sensor of the fluid and the detection electronics are typically interconnected by electrically conducting connectors e.g. wires or cables, or by optical wave guides such as optical fibres. However, such connections can be sensitive to damage and for patients or elderly confined to a bed they can be very inconvenient and put severe constraints on their movability.

Particularly for patients or elderly who are able to walk around, such sensor-detector connections cannot be used without restricting their movability.

Since lack of movability of patients and elderly requires an increased level of monitoring of e.g. wounds and urinary and faecal incontinence by personnel, such a monitoring often being considered inconvenient and cumbersome, sensor-detector connections of monitoring devices implicitly contribute to bad sanitary conditions for patients and elderly and contaminated environments of wound healing sections, incontinence sections and the like of hospitals and nursery homes.

Wireless sensor-detector connection based on suitable receiver/transmitter electronics at the sensor and detection electronics can be contemplated. However, in order to function, such electronics requires a portable power supply such as an electric battery. This is impractical because of the necessary exchange or recharge of discharged batteries.

Consequently there is a need for wireless connection of fluid sensor and detection electronics which does not require portable power supply as well as sensors suited for sensing exposure of a fluid without requiring external power supply.

Such sensors should be reliable in use and easy to mass-produce, particularly when such sensors are based on radiofrequency resonant circuit devices, these should be reliable and cheap.

The term "wireless sensor-detector connection" is intended to mean that the connection between the sensor and detector is operated with or actuated by electromagnetic waves. Specific ranges of electromagnetic waves include radiowaves or microwaves, in particular radiowaves.

Prior Art Disclosures

US 4,792,790 discloses a tag-like strip affixable to an article comprising a flexible, non-conductive substrate, at least one inductive element formed from a planar conductive path, a capacitive element formed from superimposed conductive path parts, and a dielectric interposed therebetween, the conductive paths being arranged in at least two surfaces which are superimposed by folding the substrate together forming a closed resonant circuit with the inductive element; said folded substrate avoiding contacts between conductive paths to be pressed through an insulating foil therebetween as in prior art layered structures of resonant circuits.

US 5,291,180 discloses similar LC structures of flexible insulative layers folded back on itself and having on one surface an electrically conductive layer forming multi-turn spirals in a counter turning relationship in a like folded superposition.

US 6,031,458 discloses a polymeric radio frequency resonant tag and method of its manufacture for use in detection of theft of articles for sale, said tag comprising a substrate, and first and second conductive pattern layers based on a polymeric binder and connected to form an inductive element and further connected to first and second capacitor electrodes having a dielectric film thereinbetween; said layers being sequentially applied, cross-linked and bonded together and adhered onto the substrate. The conductive pattern layers are applied using screen-printing with conductive printable ink. The dielectric film is screen-printed using e.g. a UV curable dielectric medium. The first and second conductive layers are connected through contacts via perforations in the dielectric film, specifically provided by impressing a dowel at the point of connection.

US Patent No. 4 646 066 discloses an indicator device and method of measuring incremental environmental exposure of an environmental parameter; said method comprising measuring responses of a target to an electromagnetic interrogation signal; said target including a tuned circuit and an element that is sensitive to environmental exposure, especially exposure to specified fluids e.g. liquids and water vapour, influencing the electronic or ionic conductivity. There is no teaching of parameter influences including the capacitance of the tuned circuit, nor of a measuring method based on changes of transmission of electromagnetic energy of oscillators transmitting electromagnetic energy to the tuned circuits.

## 2. DISCLOSURE OF THE INVENTION

### Object of the Invention

It is an object of the present invention to provide a radiofrequency resonant circuit device which avoids through-contacts through insulting layers between conductive layers. Further, it is the object to provide such a device which can be produced by mass production with high reproducibility of the parameters of the resonant circuit, in particular the capacitance of the capacitive element.

It is another object of the present invention to provide a method and apparatus for detecting a fluid without using any physical connection between a sensor of the fluid and an electronic detection system therefore and without requiring a portable power supply for the fluid sensor.

It is another object of the present invention to provide a method and apparatus for detecting the level of a fluid in a container, in particular the level of a human body fluid collected in a container; or for detecting the level of an infusion liquid in a container, in particular the level of an infusion liquid in a plastic bag, e.g. of soft plastic that collapses during emptying thereof.

It is another object of the present invention to provide a method and apparatus for detecting a body fluid from a human or animal body, in particular a leak of body fluid from a human suffering from e.g. urinary and/or faecal incontinence, a human or animal undergoing surgery requiring a drain of body fluid, or a human or animal having e.g. a bleeding wound.

Further objects will be apparent from the description.

### Solution According to the Invention

In an aspect of the present invention there is provided a radiofrequency resonant circuit sensing device comprising

    (a) a substrate having a surface;

    (b) a layered structure comprising:

        (i) at least one first electrically conducting means comprising an inductance (L);

        (ii) at least one second electrically conducting means; and

        (iii) at least one electrically insulating means which separates at least a part of said first electrically conducting means from said second electrically conducting means;

    either of said at least one first or second electrically conducting means being positioned on said substrate;
    the first and second electrically conducting means and the electrically insulating means being electrically connected to form a resonant circuit (LC);

wherein parts of the at least first and second electrically conducting means provide the electrodes of at least one capacitance (C), and
parts of said first and second electrically conducting means provide at least one connection between the first and second electrically conducting means, and
a sensing means for sensing a change of resonance conditions of the resonant circuit,
whereby the feature of providing at least one connection between the first and second electrically conducting means ensures that through-contacts through the electrically insulating means can be avoided.

In a preferred embodiment the at least one connection between the first and second electrically conducting means is constituted by contact between parts of said first and second electrically conducting means,

whereby it is obtained that connections of first and second electrically conducting means can be realized by contacting the two.

In a preferred embodiment the first and second electrically conducting means comprises a material selected from the group consisting of electrical conducting polymers, paints or inks,

whereby particularly cost-effective mass-production of resonant circuits can be obtained using printing techniques such as screen-printing.

In particular for applications where a large inductance value is required under conditions of small dimensions, more layers of resonant circuits can be provided.

Accordingly, in a preferred embodiment, the device further comprises more layered structures comprising more at least one first and second electrically conducting means and more at least one electrically insulating means; parts of said more first and second electrically conducting means providing contacting each other for providing at least one connection therebetween.

Generally, the sensing means for sensing a change of resonance conditions of the resonant circuit comprises any of the parameters inductance, capacitance, and resistance, or a combination thereof.

In a preferred embodiment the at least one first electrically conducting means comprises an inductive element having a coil with separated windings for receiving a fluid,

whereby the fluid may short cut the windings and change the resonance conditions.

In order to further improve the ability of the resonant circuit sensing device to sense a fluid, either the inductive element, the capacitive element, the substrate, or a combination of these can be made particular attractive to the fluid, e.g. by coating thereof with a hydrophilic material.

In a preferred embodiment the substrate is attractive to said fluid,

whereby in an embodiment of the resonant circuit having the induction element positioned on the substrate, the windings of the coil are more likely to get into contact with the fluid. If the fluid is water or urine, it may short cut the windings and deactivate the resonant circuit.

Accordingly, in a preferred embodiment, the resonant circuit is deactivated by presence of a fluid.

Some applications do not allow the influence of a fluid, e.g. water, which could produce unintended signals, e.g. in theft security systems.

Accordingly, in a preferred embodiment, the layered structure is coated with a fluid protection layer, in particular a water repellent layer.

In many applications, it is desired to be able to deactivate the resonant circuit, e.g. to authorize release of articles of sale, whereby the deactivated resonant circuit is allowed to pass the electromagnetic detection field without triggering an alarm.

For this purpose it is known to include a fuse in series in the resonant circuit.

Accordingly, in a preferred embodiment, the resonant circuit further comprises a fuse.

In another aspect of the invention there is provided a method of providing a resonant circuit sensing device comprising

(a) providing a substrate having a surface;

(b) providing a layered structure comprising:

(i) at least one first electrically conducting means comprising an inductance (L);

(ii) at least one second electrically conducting means; and

(iii) at least one electrically insulating means which separates at least a part of said first electrically conducting means from said second electrically conducting means;

either of said at least one first or second electrically conducting means being positioned on said substrate;

the first and second electrically conducting means and the electrically insulating means being electrically connected to form a resonant circuit (LC) ;

wherein parts of the at least first and second electrically conducting means are provided with electrodes of at least one capacitance (C), and

(c) bringing parts of said first and second electrically conducting means to provide at least one connection between the first and second electrically conducting means,

whereby a very cost-effective method of mass-production of resonant circuits allowing use of printing techniques such as screen-printing is provided.

According to the invention it surprisingly turns out that electrically conducting connectors between cheap, mass produced sensing devices of fluid and the detection electronics, and battery power supply of the sensor, and receiver circuits for receiving and detecting responses of resonant circuits of the sensors can be avoided.

This provides a number of advantages e.g. that the sensor of the fluid can be separated from the detection electronics for detecting changes in the sensor. This is particularly advantageous when monitoring bodies containing fluid wherein the sensor and the detection electronics used cannot be connected permanently by electrically conducting connectors, or when they can only be connected by such connectors with great difficulties or inconveniences. Also, the oscillators and detection electronics can be simplified.

"Uses of the resonant circuit sensing device for detecting a fluid"

Accordingly, in still another aspect according to the invention there is provided uses of the method or the apparatus as claimed for detection of fluid level in one or more containers, in particular incontinence containers, specifically diapers; and use for detecting whether a container containing a fluid has been emptied for the fluid in particular emptying of an infusion containing use for monitoring leak of fluid from a container, or a human or animal body, in particular from a human suffering from urinary and/or faecal incontinence; whereby the health care personnel can monitor the hygienic condition of e.g. a diaper. When a body leak has been detected proper care of e.g. changing the diaper can then be taken.

"Sensing devices of a fluid"

Still another aspect of the present invention relates to sensors usable for such methods and apparatus in sensing a fluid, more specifically it relates to sensing devices comprising resonant circuits which are responsive to parameters that are able to influence the impedance thereof, e.g. responsive to a fluid.

"Articles comprising the sensing device"

In another aspect of the present invention there is provided an article comprising the sensing device.

Preferred articles include, but are not limited to, articles for containing or for taking up fluid or for delivering fluid; such container e.g. being monitored for being filled or being emptied.

In a preferred embodiment, the article consists of a hygienic article for healthy development and maintenance of health.

In a particularly preferred embodiment, the article consists of an absorbent or a bandage in form of a wrap or a trapping used to protect, cover or immobilise an injured or diseased part of a human or an animal, or used during surgery.

In a particularly preferred embodiment, the article consists of an absorbent for urine or faeces, in particular a diaper.

"Use of articles comprising the sensing device"

Articles comprising a sensing device according to the present invention are preferably used in a method of monitoring hygienic conditions of one or more patients; said method comprising applying one or more hygienic articles according to the invention to one or more patients, each article having a sensing device with a resonant circuit that differ from each other; and transmitting electromagnetic energy to said resonant circuits.

In an embodiment, the method further comprises monitoring at least one response of said resonant circuits, thereby allowing the prior art techniques of interrogating the resonant circuit to be used.

In a preferred embodiment, the method further comprises monitoring changes of the transmission of electromagnetic energy of one or more oscillators transmitting electromagnetic energy to said resonant circuits, thereby obtaining the advantages of the method according to the present invention.

Further advantages will be apparent from the detailed description.

Sensing Devices

In its broadest aspect, the sensing device according to the present invention does not necessarily depend on the method of detecting changes of one or more characteristics of the resonant circuit, i.e. for example by detecting the change of the transmission of electromagnetic energy of the oscillators according to the invention, or by detecting a response of the resonant circuit according to prior art methods. However, the specific resonant circuit used typically depends on the parameter to be detected, e.g. a fluid.

Known methods include those referenced in US 4 646 066, e.g. US 4 321 586.

According to the aspect of the invention relating to detecting a fluid, a resonant circuit has one or more characteristics which change by contact between the resonant circuit and a fluid.

In a preferred embodiment of both aspects, the characteristics comprise resistance, capacity, inductance, or any derivative thereof.

By contact of the resonant circuit with the fluid, one or more of the characteristics resistance R, capacitance C and inductance L, or any derivative thereof, e. g. the resonance frequency

$$\omega_o = (LC)^{-\frac{1}{2}},$$

or higher harmonics thereof, or the quality factor $Q = \omega L / R$, e.g. at resonance $\omega = \omega_o$, change.

When one or more of the characteristics change, the ability of the resonant circuit to receive electromagnetic energy changes. This can be detected by detecting changes in one or more characteristics of one or more oscillators transmitting electromagnetic energy to said resonant circuit.

Generally, sensing devices can be manufactured in any suitable way that allow the external parameters to affect the impedance of the respective resonant circuits.

In the particular aspect of the invention relating to detecting a fluid, resonant circuits can be prepared in any suitable way that ensures the penetration of fluid into the resonant circuit to such an extent that one or more of its characteristics are changed.

In the particular aspect of the method of detecting a fluid, the change of characteristics of the resonant circuit causes a detectable change in one or more of the characteristics of the one or more oscillators.

In a preferred embodiment, the resonant circuit consists of a coil having separated windings which can receive the fluid and short cut the circuit.

The windings can be of any suitable material. In a preferred embodiment, the windings are made of an electrically conducting material selected from the group consisting of metals such as aluminium, copper, tin; an electrically conducting polymer such as polyaniline; and an electrically conducting polymer blend such as poly(p-phenylene vinylene), polyacrylamide, polyaniline and polyethylene, or electrically conducting silicone, or combinations of these.

Specifically useful electric conducting polymers include a flexible, crease resistant, one component, carbon filled ink and coating sold by Emerson & Cuming Speciality Polymers under the trademark Amicon C 932-74. Another useful electric conducting polymer is a highly flexible, crease resistant, one component, silver filled, ink and coating sold by Wacker-Chemie under the trademark Elastosil N 189. Both products are environmentally acceptable.

In the particularly aspect of detecting temperature changes, the resonant circuits can be prepared in any suitable way that ensures the influence of different temperatures to affect the impedance of the resonant circuit to such an extent that one or more of its characteristics are changed.

In the particular aspect of the method of detecting a temperature change, the change of the characteristics of the resonant circuit causes a detectable change in one or more of the characteristics of the one or more oscillators.

The fluid sensing device can be placed in any suitable position to detect the desired fluid. In preferred embodiments, the fluid sensing device is contained in or attached onto a container; or it is embedded in a diaper; or it is embedded in a carrier with an adhesive, such as a sticker, to be attached on a desired location, e.g. onto said container or diaper.

Several resonant circuits can be positioned in one location to encode for given patterns of frequencies, e.g. several small resonant circuits of e.g. different frequencies placed in a diaper can provide a unique identification of e.g. individual patients or elderly being monitored in a hospital or a nursing home.

In a particular aspect the present invention relates to a sensing device for sensing a fluid as claimed in claim 46 that can be mass produced by a printing process or a dispensing process.

A sensing device according to the invention has a number of advantages.

It can be produced by an application process such as a printing process or a dispensing process comprising a series of steps providing a layered structure of the first electrical conducting means, the electrical insulating means, and the second electrical conducting means. Printing or dispensing of suitable electrical conducting materials and electrical insulating materials, respectively, allows very fast and easily controllable production processes to be used.

A particular advantage is obtained, compared to prior art productions techniques of prior art resonant circuits using e.g. copper or tin as the electrical conducting means, in that a flexible resonant circuit can be produced. This is important for many applications wherein the resonant circuit is to be applied on or embedded in a carrier, e.g. a diaper, which can be bent and possible folded.

Further, sensing devices according to the present invention are advantageous in that environmentally acceptable materials and/or smaller amounts can be used thereby avoiding discharging used sensing devices including e.g. copper to the environment.

Thus, by selecting a suitable flexible substrate of a material such as paper, textile, woven, non-woven, suitable polymer, plastics, and a flexible electrical conducting polymer for the first and second electrical conducting means, which polymer can be the same or different for the two conducting means, and a suitable flexible insulating material such as polyurethane lacquer for the insulating means, flexible resonant circuits can be provided. A suitable porous material allowing penetration of humidity and/or liquids is Tyvek® supplied by DuPont.

In a preferred embodiment, the first and second electrical conducting means is composed of a material selected from the group consisting of electrical conducting polymers such as electrical conducting silicones.

The electrical insulating means is composed of any suitable material for providing electrical insulation. In a preferred embodiment the electrical insulating means consists of polyurethane laquer.

Consequently, in still a further aspect the present invention relates to a method of producing a sensing device.

In a preferred embodiment, the application process involves applying the suitable electrical insulating material onto the first electrical conducting means covering all or parts thereof, e.g. leaving parts for connecting points and areas to be exposed to the fluid during operation of the sensing device uncovered.

In a preferred embodiment, the application process is a printing process such as stencil printing and/or a dispensing process.

The term "dispensing" is intended to mean that a material, here a suitable electrical conducting material or a suitable insulating material which as dispensable, is applied to a substrate material by a suitable application means, including but not limited to application of a continuous fluid of the material, and application of droplets of the material by spraying.

Oscillators

According to the invention an oscillator operates in functional proximity of a resonant circuit.

In the present context the expression "functional proximity of a resonant circuit" is intended to mean that an oscillator in one location radiates electromagnetic energy, e.g. in form of radiowaves or microwaves, which can be fully or partly transferred to a resonant circuit in another location.

Generally, any suitable oscillator can be used, i.e. an oscillator which is able to produce electromagnetic oscillations and to emit electromagnetic energy e.g. in form of radiowaves or microwaves which is fully or partly received by the resonant circuit.

It should be understood that more than one oscillator can operate in functional proximity of said resonant circuits e.g. if more oscillators operates at different frequency ranges, and if changes in different characteristics of the resonant circuit are to be detected.

Generally, an oscillator comprises a generator which generates a high frequency signal of radiant energy e.g. an ac current or voltage, or an impulse, of a frequency around that of the resonant circuit. Generators can be any suitable generator known in the art, e. g. radio frequency signal generators.

In a preferred embodiment, the oscillator comprises a generator generating a signal of electromagnetic energy of a frequency comprising that of the resonant circuit.

An oscillator comprises one or more inductors. The inductors can be internal or external to a cabinet housing the oscillator. Preferably there is one or more inductors for each different band of operation for each resonant circuit. Preferably more inductors can be interchanged either manually or automatically. The inductors can be of any suitable form. In a preferred embodiment the inductors are external inductors in form coils.

Functional proximity between the oscillators and the resonant circuits can be achieved in any suitable way. In a preferred embodiment, the inductors are external inductors in form of coils which surrounds the resonant circuit. In a particularly preferred embodiment, the coils are embedded in a bed for monitoring leak of body fluids of a human.

In another preferred embodiment, the oscillators comprise one or more antennas whereby the direction of the electromagnetic radiation, e.g. radio waves, can be more accurately defined. An antenna can be any suitable antenna known in the art, e.g. a dipole. In a preferred embodiment, the antenna comprises a radiator element, transmission lines, and optional transformers, coupled to the inductor, or constituting a part of the inductor.

Detectors

The apparatus further comprises one or more detectors for detecting one or more changes in one or more characteristics of said oscillators upon changes in the characteristics of one or more resonant circuits.

The characteristics of the oscillators comprise any suitable characteristic.

In a preferred embodiment the characteristics of the oscillator comprise current, voltage, or a derivative thereof such as power, whereby one or more characteristics, or derivatives thereof, of the resonant circuit are detected, e.g. change in frequency, particularly the resonance frequency; change in quality factor; and wholly or partial suppression or restoration of any of these.

In a preferred embodiment one or more of the detectors detect an increase or a decrease of energy loss of one or more of the oscillators.

Further, preferred embodiments of the invention and uses appear from the following detailed description and claims.

3. BRIEF DESCRIPTION OF THE DRAWINGS

In the following, the invention is further disclosed with detailed description of preferred embodiments, reference being made to the drawings in which

FIGS. 1A and 1B show a preferred embodiment of a sensing device according to the invention for sensing a fluid;

FIGS. 2A-2C and 3A-3C show a preferred embodiment of a method for the production of a sensing device according to the invention;

FIGS. 4A-4C show another preferred embodiment of a method of producing another sensing device according to the invention;

FIGS. 5A-5C show applications of the sensing device shown in FIG. 1 wherein fluid droplets affects the resonant circuit without penetrating the electrical conducting means (FIG. 5A), or with penetration thereof (FIG. 5B);

FIGS. 7A-7C and FIGS. 8A-8C are similar to FIGS. 2A-2C and FIGS. 3A-3C;

FIGS. 9A and 9B show adjustment of the position of first and second flat extended conductors;

FIGS. 10A and 10B illustrate diaper production;

FIG. 11 illustrates production of resonant circuits using single step application;

FIGS. 12A and 12B and FIGS. 13A and 13B show a resonant circuit sensor coated partly with a fluid protective layer;

FIGS. 14, 15, and 16A and 16B show different embodiments of applications of the resonant circuit; and

FIGS. 17A and 17B and FIGS. 18A and 18B show applications of the resonant circuit inside and outside a collection bag and an infusion bag, respectively.

4. DETAILED DESCRIPTION

Embodiment of sensing devices according to the invention

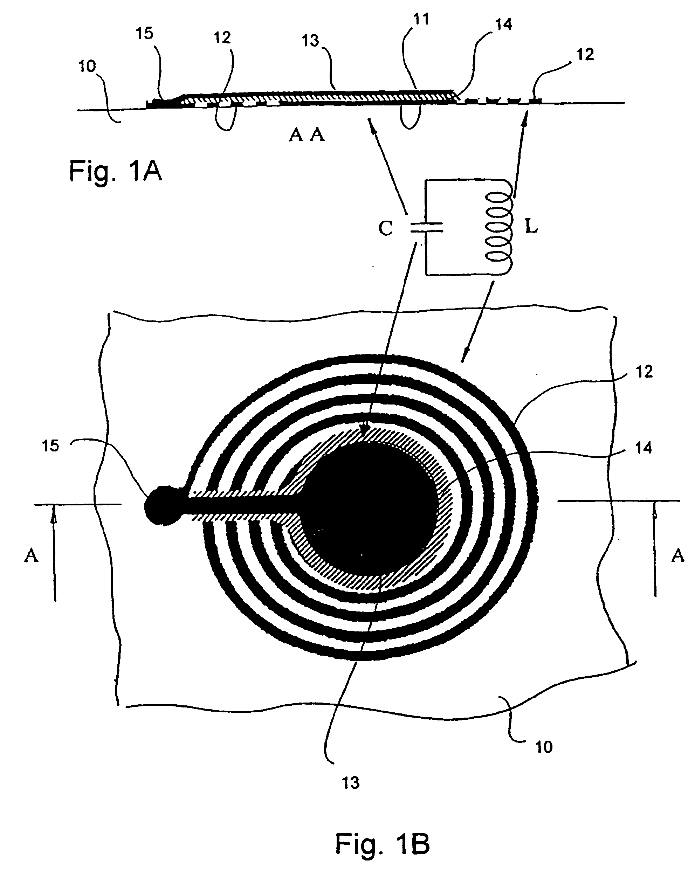

FIGS. 1A and 1B show a preferred embodiment of a sensing device for sensing a fluid according to the invention.

It comprises a substrate having a surface, here exemplified by a flexible material 10 such as paper, textile, plastic, etc. in the form of e.g. a sheet or a foil.

On its surface opposite to the layered structure, or on any other suitable position, the substrate can have an adhesive layer whereby the sensing device can be adhered to the surface of e.g. a diaper, like a sticker, a label or a tag.

At least one first electrical conducting means comprising an inductance (L), here exemplified by a winding or a coil 12 of a conducting polymer such as an electrical conducting silicone of Wacker Silicones e.g. Semicosil 970 from Wacker-Chemie, and typically in form of a flat winding but not limited thereto, which inductance is positioned on the substrate surface, and parts thereof 11, here exemplified by a first extended conductor which provides a first electrode of at least one capacitance (C) and parts thereof, here a connecting point 15 which provides a connection to at least one second electrical conducting means, here exemplified by a second flat extended conductor 13 which provides a second electrode of said at least one capacitance and a flat connecting conductor extending from the second flat extended conductor to the connecting point.

At least one electrical insulating means, here an electrical insulating layer 14, separates at least a part of said first conducting means from said second conducting means, here the flat extended conductor and the flat connecting conductor are insulated from the windings and the first extended conductor.

The first and second electrical conducting means and the electrical insulating means are electrically connected to form a resonant circuit, the equivalence diagramme (LC) of which is shown in FIGS. 1A and 1B.

It should be understood that a resonant circuit of a physical sensing device deviates from the idealized equivalence diagramme of FIG. 1, mainly due to loss factors, such as resistive losses in the leads, energy lost from the circuit by radiation etc. Therefore, the actual performance of the circuit is more closely approximated by including a resistor in the LC equivalence diagramme (not shown in FIG. 1). Also, a dedicated resistor may be included in the circuit in order to modify the circuit performance in a particular fashion. The resistor, whether it is merely a representation of loss factors of it signifies a combination of loss factors together with one or more dedicated resistors, may be coupled in parallel, or in series, or both, with the other components of the resonant circuit. Determining suitable values of the components in the equivalence diagramme and of the resistor is considered to lie within the capabilities of those skilled in the art.

It should be noted that the term "being positioned on the surface of said substrate" is intended to include any such positioning wherein the material of the at least one first or second electrical conducting means penetrates wholly or partly into the substrate.

Principle of Design

The method of manufacturing a response circuit provided by the present invention enables an extensive range of options of size and arrangement of device. The invention allows a range of printing techniques to be applied in the method including continuously rotating printing methods for printing the conductive and dielectric patterns.

Generally, there is no requirement for a separate mechanical or other process for making the connection between the individual conductive layers. The design enables the printing process itself to connect the two conductive layers at the required position.

The design minimizes the size of the capacitor dielectric which is only printed where layers need to be separated.

Many conductive layers may be printed on top of each other using simple printing techniques without recourse to secondary processes for completing the device.

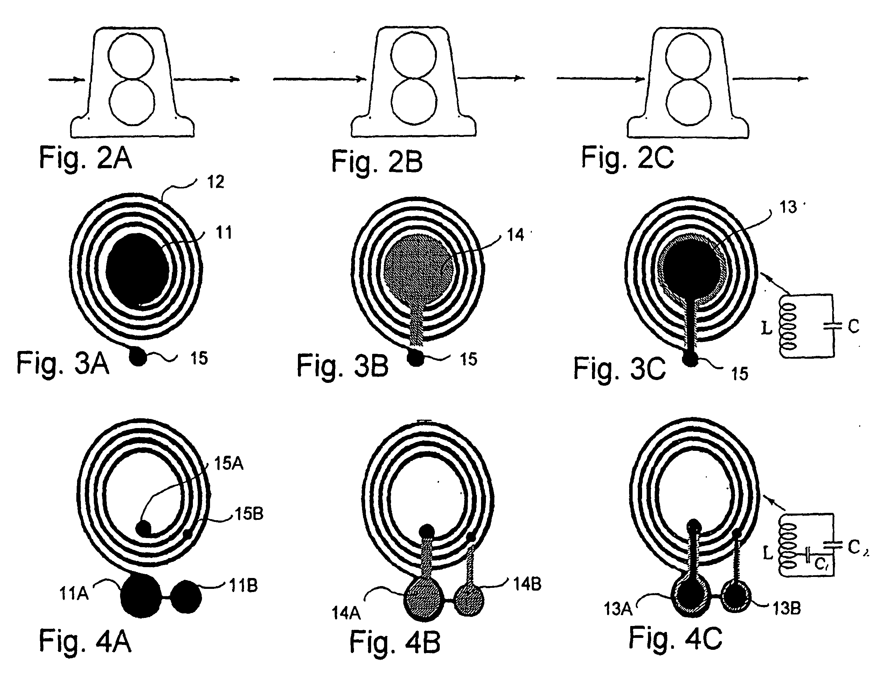

FIGS. 2A-2C and 3A-3C show a preferred embodiment of a method for the production of a sensing device according to the invention.

FIGS. 2A-2C illustrates three consecutive steps of application of the at least one first electrical conducting means 11, 12, 15 (FIG. 3A), the at least one second electrical conducting means 13, 15 (FIG. 3C) and the at least one electrical insulating means 14 (FIG. 3B), here exemplified by a printing process. The respective layers are shown in FIGS. 3A-3C.

FIG. 3A shows the first conductive pattern to be printed. Item 11 is the first layer of the capacitor, item 12 is the coil and position 15 is the location for the contact to the second layer of the capacitor. This same location would be used for contact to any subsequent layers of the conductive patterns. In this instance, this location is positioned outside of the coil.

FIG. 3C shows the second conductive layer of capacitor, item 13, printed on top of the dielectric mask.

This second layer makes the connection for each side of the capacitor at location 15.

This procedure may be repeated providing as many layers as necessary.

FIGS. 4A, 4B and 4C illustrate a variation providing a response circuit with a double capacitor and coil combination. This variation has the connection between the conductive patterns located both inside and within the coil at locations 15A and 15B. The two capacitors are formed from conductive areas 11A and B, dielectric masks 14A and B and conductive layers 13A and B.

FIGS. 4A-4C show another preferred embodiment of the method of producing a sensing device according to the invention wherein it is illustrated that more complex resonant circuits, e.g. including two capacitances $C_1$ and $C_2$, can be provided. The substrate is not shown.

The first electrical conducting means includes two extended conductors 11A, 11B, and two connecting points 15A, 15B (FIG. 4A). The two extended conductors are then covered by respective electrical insulating layers 14A, 14B (FIG. 4B). Finally, the second electrical conducting means in form of two extended conductors 13A, 13B are applied on the electrical insulating layers.

Examples of suitable application apparatus to be used in carrying out the method of producing a sensing device according to the invention are screen printers and stencil printers from e.g. EKRA, Eduard kraft GmbH Maschinen Fabrik, Bönnigheim, Germany. Specific apparatus can be mentioned e.g. E1™ Semi-automative Screen Printer and M2™ Semi-automatic Thick-Film Screen Printer, the latter being particularly suited for printing of solder pastes, through-hole coating using vacuum techniques, and multilayer systems. Further suitable apparatus are dispensing apparatus from e.g. Asymtek.

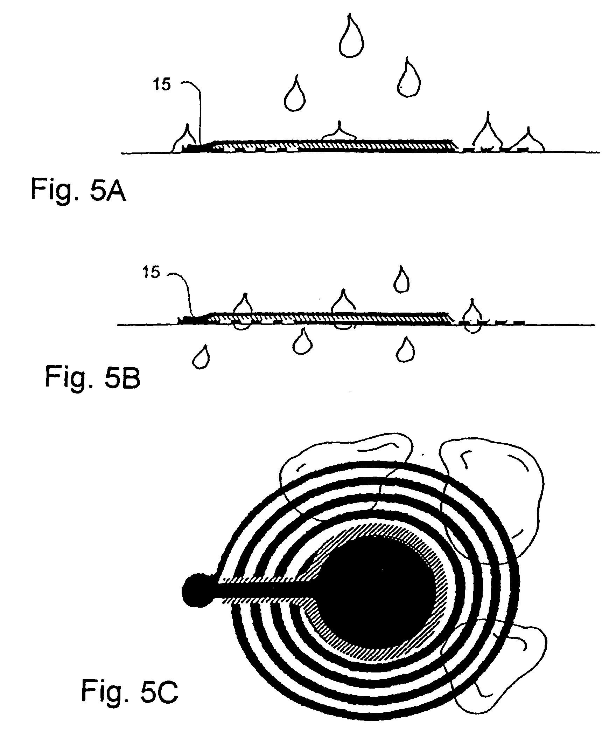

FIGS. 5A-5C show an application of the sensing device wherein fluid droplets affects the resonant circuit without penetrating therethrough (FIG. 5A), and with penetration (FIG. 5B). FIG. 5C provides a top view.

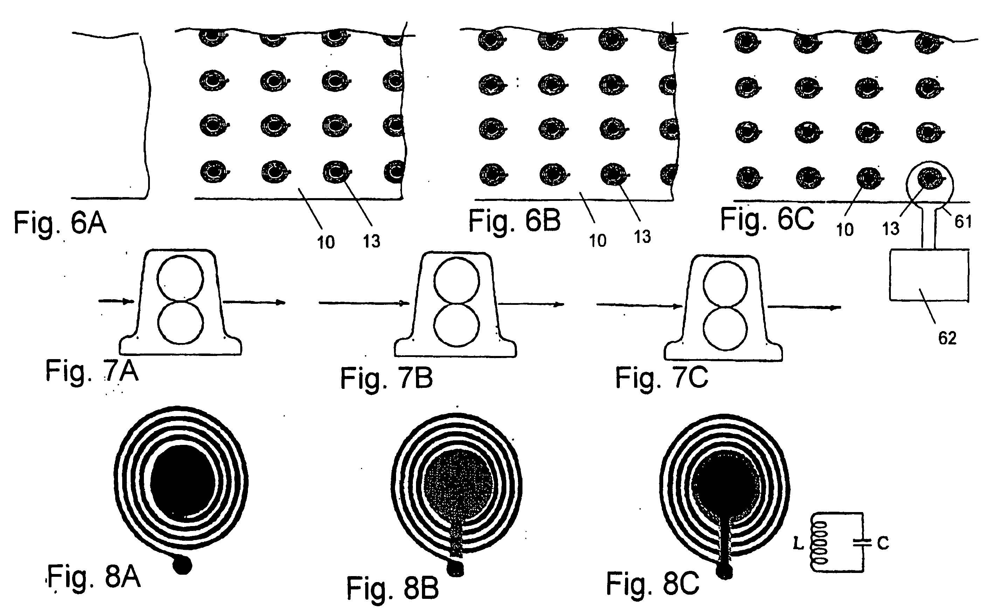

FIGS. 6-8 illustrate that more resonant circuits can be produced in parallel.

FIGS. 7A-7C and 8A-8C are similar to FIGS. 2A-2C and 3A-3C.

FIGS. 6A-6C illustrate production of resonant circuits 13 in parallel process lines on a single continuous foil 10.

In FIG. 6C, there is illustrated means to assist in controlling the printing process, here illustrated by an antenna 61 positioned above a resonant circuit 13 and connected to a control unit 62. The control unit 62 feeds control signal to the application apparatus in the various process steps for adjusting e.g. the speed of the foil, amounts and speed of applied layers, based on the measured quantities of the resonant circuit.

General control test parameters include the frequency of the resonant circuit, but other parameters such as the Q-value or frequency dependent impedance can be contemplated for use in control purposes.

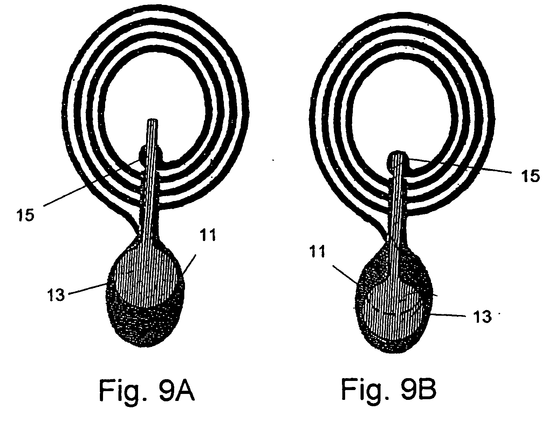

FIGS. 9A and 9B illustrate the adjustment of the position of the first and second electrically connecting means, here the first 11 and second 13 flat extended conductors providing electrodes of the capacitive element and the connecting point 15 are moved with respect to each other. In FIG. 9A, the two electrodes fully overlap whereas they are displaced with respect to each other in FIG. 9B.

The control parameter measured for controlling this type of adjustment typically includes the capacitance of the resonant circuit, e.g. measured by the Q-value, because the degree of overlap of the capacitor electrodes is changed significantly.

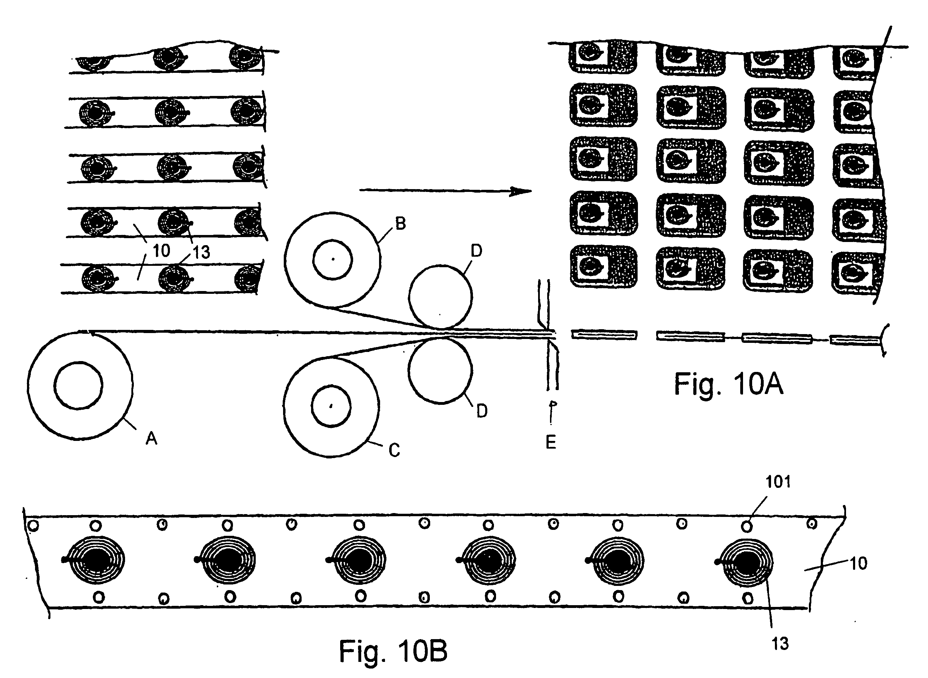

FIGS. 10A and 10B illustrate diaper production wherein several parallel resonant circuits 13 on separate foils 10, they could also be positioned on the same foil, are fed from a drum A and laminate on both sides by diaper compatible materials fed from drums B and C and controlled by compression rollers D. The actual diaper produced may include other materials not shown, e. g. water absorbents and water repellent front cover foils. At E, individual sections are punched out.

In another application, the same set up can be used to produce resonant circuit tags for theft security of sales products, in such products the resonant circuit is usually laminated with water repellent foils.

FIG. 10B illustrates perforations 101 in the foil 10 for controlling the movement thereof.

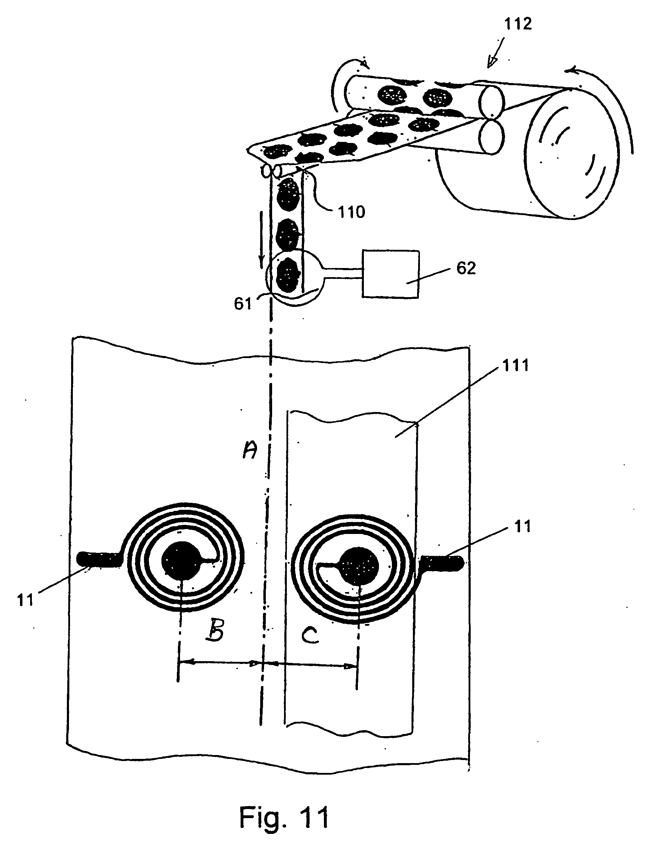

FIG. 11 illustrates the production of resonant circuits using a single step application apparatus 112 of pairs of resonant circuits B,C according to the invention wherein the conductive layers, here connecting points 11, are brought into contact with each other by folding the substrate about the folding line A by means of folding guide 110. Between the parts to be folded against each other there is inserted an insulating intermediate layer 111.

An antenna 61 and control unit 62 are positioned of the folding step and function to control the folding process in providing the desired contact between the two conductors.

In an embodiment, curement of the conductive UV-curable layers is obtained by UV-irradiation of the folded foil with the connecting points of the conductive paths in contact thereby providing fixed contacts.

In another embodiment, not shown, the connecting point 11 is placed closer to the folding line thereby providing a hinge-type contact which is useful in applications where the intermediate layer is constituted by available capacitive element e.g. a plastic bag. The hinge-type resonant circuit is able to sense whether the bag has a content or not or the bag is being filled or emptied.

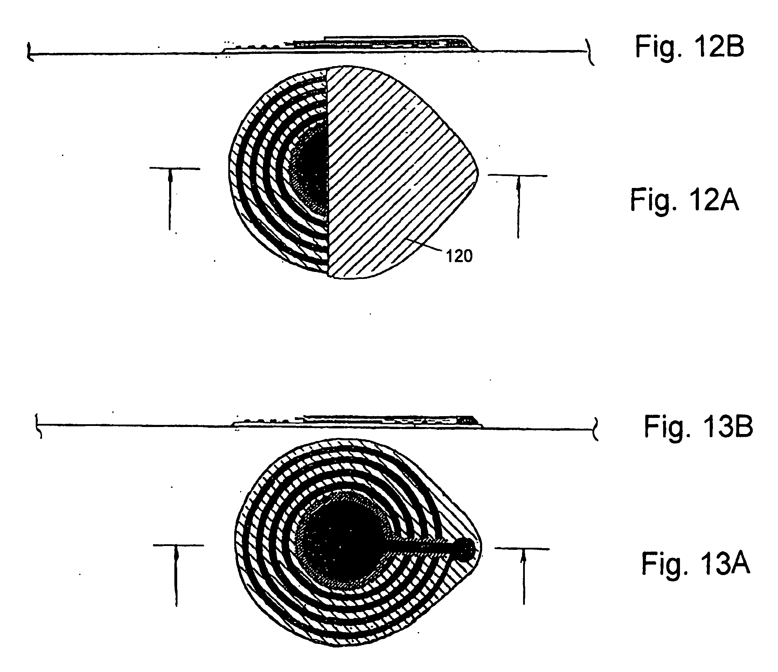

FIGS. 12A and 12B and FIGS. 13A and 13B illustrate means to modify the sensitivity to a fluid of the resonant circuit sensor by coating with a fluid protective layer 120.

In FIG. 12A, the protective layer covers fully the one side of the resonant circuit and covers partially the other side.

In FIG. 13A, only one side is covered.

In another embodiment, the fluid protective layer can be a fluid attracting layer to enhance the sensitivity to fluids.

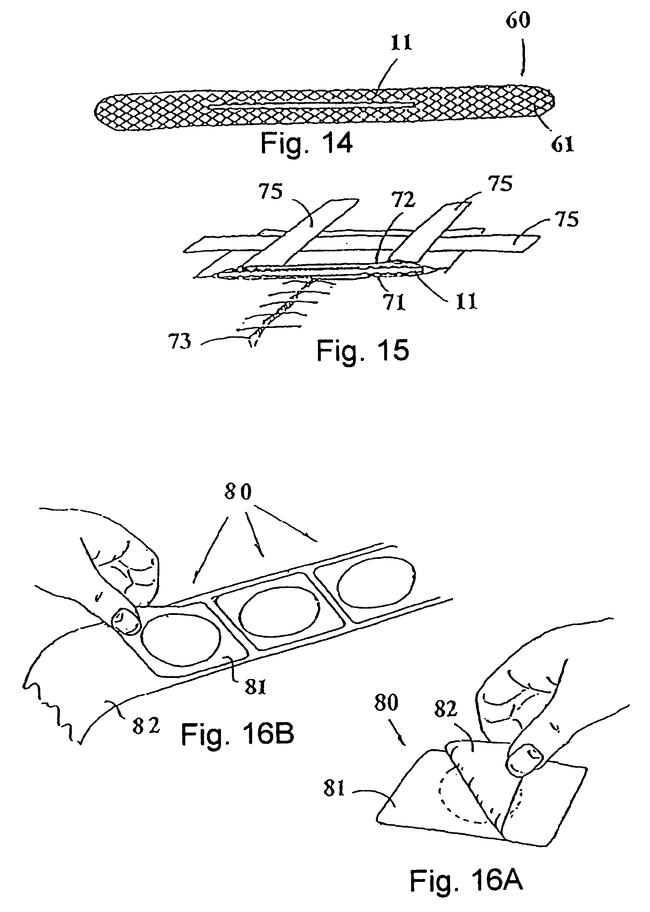

FIGS. 14, 15, and 16A and 16B show different embodiments of applications of the resonant circuit, in particular for a sensing device according to the present invention.

FIG. 14 shows a resonant circuit 11 embedded in a diaper or bandage 60 able to absorb the fluid, e.g. urine or a body fluid, e.g. blood from a wound, by an absorbing material 61.

FIG. 15 shows a laminated resonant circuit 11 having the resonant circuit 11 embedded between two materials 71, 72 or which one 71 is compatible with human skin and allows humidity and body fluid to penetrate. The laminated circuit can be applied directly on the skin wholly or partly covering a bleeding wound 73. It can be fixed to the skin either by means of a plaster or a tape 75.

FIG. 16A shows an attachable resonant circuit 80 of the type shown in FIG. 15 wherein the resonant circuit is embedded between a carrier material wholly or partly covered with adhesive, e.g. a skin compatible adhesive, material 81 for affixing the resonant circuit to e. g. the skin, or a diaper, and a releasable cover material 82 covering said adhesive properties thereof before use. It should be noted that the resonant circuit can be of the laminated type, or it can be directly adhered to or incorporated in the carrier material.

FIG. 16B shows several attachable resonant circuits 80 similar to that of FIG. 16A provided on an "endless" releasably cover material 81, particularly useful for fast and easy handling and application of many sensing devices, e.g. fluid, to a human body or an article, e.g. a diaper.

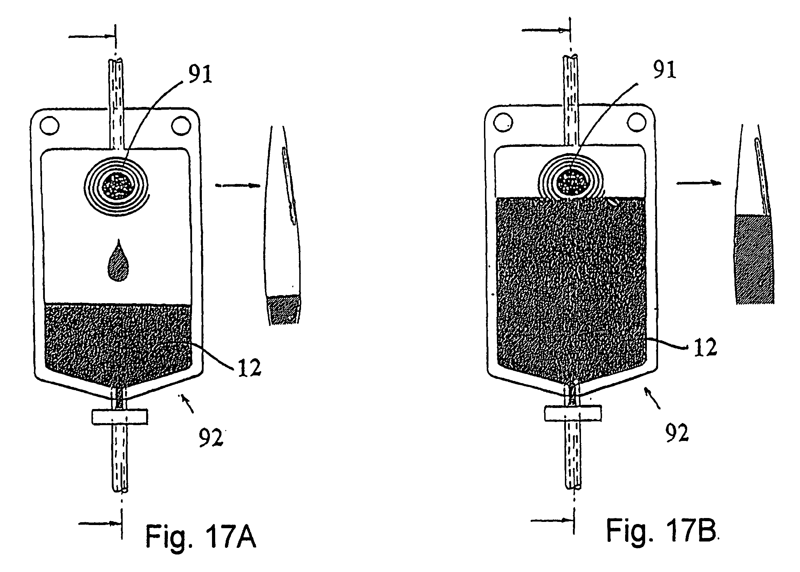

FIGS. 17A and 17B with cross sectional views show application of a fluid sensing device, i.e. a resonant circuit 91 e.g. of the type shown in FIG. 5C or FIG. 15, contained in a container 92, e.g. a collection bag, or drain bag, for monitoring the level of the collected fluid, e.g. drain fluid 12. The characteristics of the resonant circuit are changed upon contact with the fluid, see FIG. 17B.

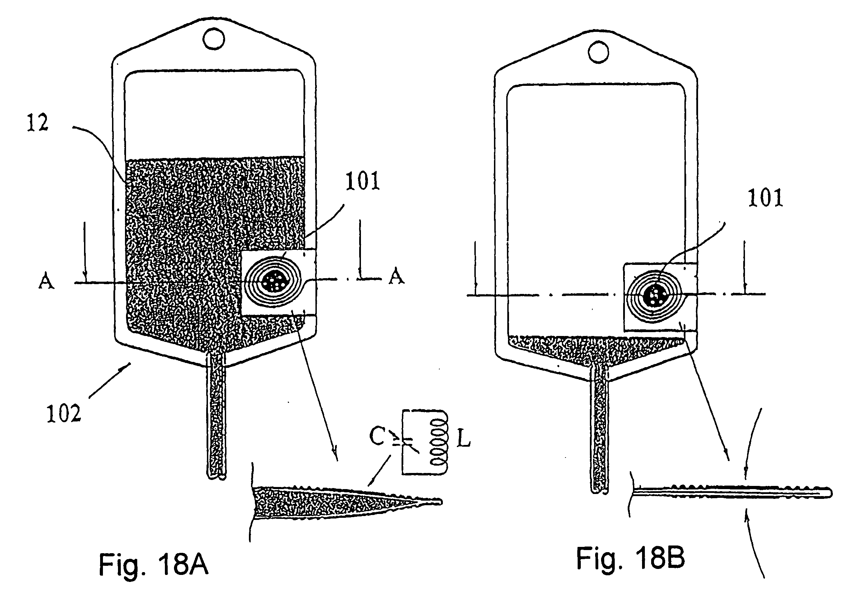

FIGS. 18A and 18B show another application of a resonant circuit 101, e.g. of the type which can be used in combination with a hinge-type resonant circuit according to an embodiment discussed with respect to FIG. 11, attached to the outside of a container 102, e.g. an infusion bag, at the fluid level A-A for monitoring the level of a fluid 12 contained therein, when the level of the fluid sinks below the level A-A, the ability of the resonant circuit to receive electromagnetic energy from an oscillator changes because the capacitor between the serially connected coils changes as the container sides collapse against each other. This embodiment can be used to monitor infusion liquids and indicate an alarm, when the infusion bag is empty.

## Claims

1. A radiofrequency resonant circuit sensing device comprising

   (a) a substrate (10) having a surface;

   (b) a layered structure comprising:

   (i) at least one first electrically conducting means (11,12) comprising an inductance (L);

   (ii) at least one second electrically conducting means (13); and

   (iii) at least one electrically insulating means (14) which separates at least a part of said first electrically conducting means from said second electrically conducting means;

   either of said at least one first or second electrically conducting means being positioned on said substrate;
   the first and second electrically conducting means and the electrically insulating means being electrically connected to form a resonant circuit (LC);
   wherein parts (11,13) of the first and second electrically conducting means provide the electrodes of at least one capacitance (C), and
   parts (15) of said first and second electrically conducting means provide at least one connection between the first and second electrically conducting means, and
   a sensing means for sensing a change of resonance conditions of the resonant circuit in response to a fluid.

2. A device according to claim 1 wherein the at least one connection between the first and second electrically conducting means is constituted by contacts between parts (15) of said first and second electrically conducting means.

3. A device according to claims 1 or 2 wherein the first and second electrically conducting means comprises a material selected from the group consisting of electrical conducting polymers, paints or inks.

4. A device according to claims 1-3 further comprising more layered structures comprising more at least one first and second electrically conducting means and more at least one electrically insulating means; parts of said more first and second electrically conducting means providing contacts with each other for providing at least one connection therebetween.

5. A device according to claims 1-4 wherein the at least one first electrically conducting means comprises an inductive element having a coil with separated windings for receiving a fluid.

6. A device according to claims 1-5 wherein the substrate is attractive to said fluid.

7. A device according to claims 1-6 wherein the layered structure is coated with a fluid protection layer.

8. A device according to claims 1-7 wherein the resonant circuit is deactivated by presence of a fluid.

9. A device according to claims 1-8 wherein the resonant circuit further comprises a fuse.

10. A method of producing a resonant circuit sensing device comprising

    (a) providing a substrate (10) having a surface;

    (b) providing a layered structure comprising:

        (i) at least one first electrically conducting means (11,12) comprising an inductance (L);

        (ii) at least one second electrically conducting means (13); and

        (iii) at least one electrically insulating means (14) which separates at least a part of said first electrically conducting means from said second electrically conducting means;

    either of said at least one first or second electrically conducting means being positioned on said substrate;
the first and second electrically conducting means and the electrically insulating means being electrically connected to form a resonant circuit (LC);
wherein parts (11,13) of the at least first and second electrically conducting means are provided with electrodes of at least one capacitance (C), and

    (c) bringing parts (15) of said first and second electrically conducting means to provide at least one connection between the first and second electrically conducting means;

said resonant circuit being responsive to a fluid.

11. A method according to claim 10 wherein the at least one connection between the first and second electrically conducting means is established contacting parts (15) of said first and second electrically conducting means with each other.

12. A method according to claims 10 or 11 wherein the first and second electrically conducting means comprises a material selected from the group consisting of electrical conducting polymers, paints or inks.

13. A method according to claims 10-12 further comprising providing more layered structures comprising more at least one first and second electrically conducting means and more at least one electrically insulating means; contacting parts of said more first and second electrically conducting means with each other for providing at least one connection therebetween.

14. A method according to claims 10-13 wherein the layered structure is provided by printing.

15. A method according to claims 10-14 wherein the printing is controlled by monitoring resonant circuit parameters the produced resonant circuits on line and adjusting the printing process conditions to obtain desired parameters therefor.

16. An apparatus for detecting a fluid comprising: one or more oscillators (10) for transmitting electromagnetic energy; one or more resonant circuits (11) for receiving electromagnetic energy from said oscillators; and one or more detectors for detecting changes in one or more characteristics of the one or more oscillators upon changes in characteristics of the resonant circuits by contact thereof with the fluid wherein the resonant circuit is according to claims 1-9.

17. Apparatus according to claim 16, wherein the one or more resonant circuits comprise coils having separated windings for receiving the fluid and short cut the circuit.

18. Apparatus according to claims 16 or 17, wherein the windings are made of an electrically conducting material selected from the group consisting of electrically conducting polymers such as polyaniline; or electrically conducting polymer blends such as blends of poly(p-phenylene vinylene), polyacrylamide, polyaniline and polyethylene.

19. Apparatus according to claims 16-18, wherein the one or more resonant circuits are contained in or attached onto one or more containers.

**20.** Apparatus according to claims 16-19, wherein the one or more resonant circuits are embedded in a diaper.

**21.** Apparatus according to claims 16-20, wherein the one or more resonant circuits are embedded in a carrier with an adhesive such as a sticker.

**22.** Use of an apparatus according to claims 16-21 for detecting the level of fluid in one or more containers.

**23.** Use as in claim 22, wherein the containers comprise containers and infusion containers.

**24.** Use of an apparatus according to claims 16-21 for detecting whether a container containing a fluid has been emptied for the fluid or an empty container has been filled.

**25.** Use as claimed in claim 24, wherein the container comprises an infusion container.

**26.** Use of an apparatus according to claims 16-21 for monitoring a leak of fluid from a container.

**27.** Use of an apparatus according to claims 16-21 for monitoring a leak from or a supply of fluid to a human or animal body.

**28.** Use as claimed in claim 27, wherein the leak of fluid is a fluid from a human suffering from urinary and/ or faecal incontinence.

**29.** An article comprising a sensing device as claimed in claims 1-9, or a sensing device produced according to the method as claimed in claims 10-15, for containing or for taking up or for delivering of fluid.

**30.** An article comprising a sensing device as claimed in claims 1-9, or a sensing device produced according to the method as claimed in claims 10-15, for healthy development and maintenance of health.

**31.** An article according to claim 30 consisting of an absorbent or a bandage in form of a wrap or a trapping used to protect, cover or immobilise an injured or diseased part of a human or an animal, or used during surgery.

**32.** An article according to claim 31 consisting of an absorbent for urine or faeces, in particular a diaper.

**Patentansprüche**

**1.** Hochfrequenzschwingkreis-Sensorvorrichtung umfassend

(a) ein eine Oberfläche aufweisendes Substrat (10);
(b) eine geschichtete Struktur umfassend:

(i) mindestens ein erstes elektrisch leitendes Mittel (11, 12), das eine Induktivität (L) umfasst;
(ii) mindestens ein zweites elektrisch leitendes Mittel (13); und
(iii) mindestens ein elektrisch isolierendes Mittel (14), welches mindestens einen Teil des besagten ersten elektrisch leitenden Mittels vom besagten zweiten elektrisch leitenden Mittel trennt;

wobei einer der beiden besagten mindestens einen ersten oder zweiten elektrisch leitenden Mitteln auf dem besagten Substrat angeordnet ist;
wobei das erste und zweite elektrisch leitende Mittel und das elektrisch isolierende Material elektrisch verbunden sind, um einen Schwingkreis (LC) zu bilden;
wobei Teile (11, 13) der ersten und zweiten elektrisch leitenden Mittel die Elektroden mindestens einer Kapazität (C) bereitstellen, und
Teile (15) der besagten ersten und zweiten elektrisch leitenden Mittel mindestens eine Verbindung zwischen dem ersten und zweiten elektrisch leitenden Mittel bereitstellen, und
ein Sensormittel zum Erfassen einer Änderung der Resonanzbedingungen des Schwingkreises als Reaktion auf ein Fluid.

**2.** Vorrichtung nach Anspruch 1, wobei die mindestens eine Verbindung zwischen den ersten und zweiten elektrisch leitenden Mitteln durch Kontakte zwischen Teilen (15) der besagten ersten und zweiten elektrisch leitenden Mittel gebildet ist.

**3.** Vorrichtung nach einem der Ansprüche 1 oder 2, wobei das erste und zweite elektrisch leitende Mittel ein Material umfasst, welches aus der Gruppe bestehend aus elektrisch leitenden Polymeren, Lakken oder Tinten ausgewählt ist.

**4.** Vorrichtung nach einem der Ansprüche 1 bis 3 weiter umfassend weitere geschichtete Strukturen, die weitere mindestens ein erstes und zweites elektrisch leitendes Mittel und weitere mindestens ein elektrisch isolierendes Mittel umfassen; Teile welcher besagten weiteren ersten und zweiten elektrisch leitenden Mittel Kontakte zueinander bereitstellen, um mindestens eine Verbindung zwischen ihnen bereitzustellen.

**5.** Vorrichtung nach einem der Ansprüche 1 bis 4, wobei das mindestens eine erste elektrisch leitende Mittel ein induktives Element umfasst, das eine Spule mit getrennten Wicklungen zum Aufnehmen eines Fluids umfasst.

6. Vorrichtung nach einem der Ansprüche 1 bis 5, wobei das Substrat anziehend für besagtes Fluid ist.

7. Vorrichtung nach einem der Ansprüche 1 bis 6, wobei die geschichtete Struktur mit einer Fluidschutzschicht überzogen ist.

8. Vorrichtung nach einem der Ansprüche 1 bis 7, wobei der Schwingkreis durch die Anwesenheit eines Fluids deaktiviert wird.

9. Vorrichtung nach einem der Ansprüche 1 bis 8, wobei der Schwingkreis weiter eine Sicherung umfasst.

10. Verfahren zur Herstellung einer Hochfrequenzschwingkreis-Sensorvorrichtung umfassend

(a) ein Breitstellen eines eine Oberfläche aufweisenden Substrats (10);
(b) ein Bereitstellen einer geschichteten Struktur umfassend:

(i) mindestens ein erstes elektrisch leitendes Mittel (11, 12), das eine Induktivität (L) umfasst;
(ii) mindestens ein zweites elektrisch leitendes Mittel (13); und
(iii) mindestens ein elektrisch isolierendes Mittel (14), welches mindestens einen Teil des besagten ersten elektrisch leitenden Mittels vom besagten zweiten elektrisch leitenden Mittel trennt;

wobei eines der beiden besagten mindestens einen ersten oder zweiten elektrisch leitenden Mitteln auf dem besagten Substrat angeordnet ist;
wobei das erste und zweite elektrisch leitenden Mittel und das elektrisch isolierende Material elektrisch verbunden sind, um einen Schwingkreis (LC) zu bilden;
wobei Teile (11, 13) der mindestens ersten und zweiten elektrisch leitenden Mittel mit Elektroden mindestens einer Kapazität (C) versehen sind, und
(c) ein Veranlassen von Teilen (15) der besagten ersten und zweiten elektrisch leitenden Mittel mindestens eine Verbindung zwischen den ersten und zweiten elektrisch leitenden Mitteln bereitzustellen; welcher besagte Schwingkreis auf ein Fluid reagiert.

11. Verfahren nach Anspruch 10, wobei die mindestens eine Verbindung zwischen den ersten und zweiten elektrisch leitenden Mitteln hergestellt wird, indem Teile (15) der besagten ersten und zweiten elektrisch leitenden Mittel einander kontaktieren.

12. Verfahren nach einem der Ansprüche 10 oder 11, wobei das erste und zweite elektrisch leitende Mittel ein Material umfasst, welches aus der Gruppe bestehend aus elektrisch leitenden Polymeren, Lakken oder Tinten ausgewählt ist.

13. Verfahren nach einem der Ansprüche 10 bis 12 weiter umfassend ein Bereitstellen weiterer geschichteter Strukturen, die weitere mindestens ein erstes und zweites elektrisch leitendes Mittel und weitere mindestens ein elektrisch isolierendes Mittel umfassen; ein einander Kontaktieren von Teilen der besagten weiteren ersten und zweiten elektrisch leitenden Mittel, um mindestens eine Verbindung zwischen ihnen bereitzustellen.

14. Verfahren nach einem der Ansprüche 10 bis 13, wobei die geschichtete Struktur durch Drucken bereitgestellt wird.

15. Verfahren nach einem der Ansprüche 10 bis 14, wobei das Drucken kontrolliert wird durch online Überwachen der Schwingkreisparameter der erzeugten Schwingkreise und durch Anpassen der Druckprozessbedingungen, um gewünschte Parameter davon zu erhalten.

16. Gerät zum Detektieren eines Fluids umfassend: einen oder mehrere Oszillatoren (10) zur Übermittlung elektromagnetischer Energie; einen oder mehrere Schwingkreise (11) zum Aufnehmen von elektromagnetischer Energie der besagten Oszillatoren; und einen oder mehrere Detektoren zum Detektieren von Änderungen einer oder mehrerer Charakteristiken der einen oder mehreren Oszillatoren auf Änderungen in Charakteristiken der Schwingkreise durch deren Kontakt mit dem Fluid, wobei der Schwingkreis nach Anspruch 1 bis 9 ausgebildet ist.

17. Gerät nach Anspruch 16, wobei die einen oder mehreren Schwingkreise Spulen umfassen, welche getrennten Wicklungen zum Aufnehmen des Fluids und zum Kurzschliessen des Kreises aufweisen.

18. Gerät nach einem der Ansprüche 16 oder 17, wobei die Wicklungen aus einem elektrisch leitenden Material hergestellt sind, ausgewählt aus der Gruppe bestehend aus elektrisch leitenden Polymeren wie Polyanilin; oder elektrisch leitenden Polymergemischen wie Gemischen von Poly(p-Phenylvinyl), Polyacrylamid, Polyanilin und Polyethylen.

19. Gerät nach einem der Ansprüche 16 bis 18, wobei die einen oder mehreren Schwingkreise in einem oder mehreren Behältern enthalten oder daran angeheftet sind.

20. Gerät nach einem der Ansprüche 16 bis 19, wobei die einen oder mehreren Schwingkreise in eine Windel eingebettet sind.

21. Gerät nach einem der Ansprüche 16 bis 20, wobei die einen oder mehreren Schwingkreise eingebettet sind in einen Träger mit einem Klebstoff, wie ein Kleber.

22. Verwendung eines Geräts nach einem der Ansprüche 16 bis 21 zum Detektieren des Fluidniveaus in einem oder mehreren Behältern.

23. Verwendung nach Anspruch 22, wobei die Behälter Behälter und Infusionsbehälter umfassen.

24. Verwendung eines Geräts nach einem der Ansprüche 16 bis 21 zum Detektieren, ob ein Behälter, der ein Fluid enthält, von dem Fluid geleert wurde oder ein leerer Behälter gefiillt wurde.

25. Verwendung nach Anspruch 24, wobei der Behälter einen Infusionsbehälter umfasst.

26. Verwendung eines Geräts nach einem der Ansprüche 16 bis 21 zum Überwachen eines Austretens eines Fluids aus einem Behälter.

27. Verwendung eines Geräts nach einem der Ansprüche 16 bis 21 zum Überwachen eines Austretens von Fluid aus einem oder eines Einspeisens von Fluid in einen menschlichen oder tierischen Körper.

28. Verwendung nach Anspruch 27, wobei das Austreten des Fluids ein Fluid eines Menschen ist, der an einer Urin- und/oder einer Fäkalinkontinenz leidet.

29. Ein Artikel umfassend eine Sensorvorrichtung nach einem der Ansprüche 1 bis 9 oder eine Sensorvorrichtung hergestellt gemäss dem Verfahren nach einem der Ansprüche 10 bis 15 zum Enthalten, Aufnehmen oder Abgeben von Fluid.

30. Ein Artikel umfassend eine Sensorvorrichtung nach einem der Ansprüche 1 bis 9 oder eine Sensorvorrichtung hergestellt gemäss dem Verfahren nach einem der Ansprüche 10 bis 15 zur gesunden Entwicklung und Erhaltung der Gesundheit.

31. Artikel nach Anspruch 30 bestehend aus einem Absorptionsmittel oder einer Bandage in Form einer Umwicklung oder einem Auffangmittel verwendet um einen verletzten oder kranken Teil eines menschlichen oder tierischen Körpers zu schützen, zu bedecken oder zu immobilisieren, oder verwendet in der Chirurgie.

32. Artikel nach Anspruch 31 bestehend aus einem Absorptionsmittel für Urin oder, Fäkalien, insbesondere einer Windel.

**Revendications**

1. Dispositif de détection par circuit résonnant haute fréquence, comprenant :

    (a) un substrat (10) comportant une surface ;
    (b) une structure en couches comprenant :

        (i) au moins un premier moyen électriquement conducteur (11, 12) comprenant une inductance (L) ;
        (ii) au moins un deuxième moyen électriquement conducteur (13) ; et
        (iii) au moins un moyen électriquement isolant (14) qui sépare au moins une partie dudit premier moyen électriquement conducteur dudit deuxième moyen électriquement conducteur ;

    l'un ou l'autre desdits au moins un premier ou deuxième moyen électriquement conducteur étant placé sur ledit substrat ;
    les premier et deuxième moyens électriquement conducteurs et le moyen électriquement isolant étant connectés électriquement pour former un circuit résonnant (LC) ;
    dans lequel des parties (11, 13) desdits premier et deuxième moyens électriquement conducteurs fournissant les électrodes d'au moins une capacité (C), et
    des parties (15) desdits premier et deuxième moyens électriquement conducteurs fournissant au moins une connexion entre les premier et deuxième moyens électriquement conducteurs, et
    un moyen de détection pour détecter une variation de conditions de résonance du circuit résonnant en réaction à un fluide.

2. Dispositif selon la revendication 1, dans lequel ladite au moins une connexion entre les premier et deuxième moyens électriquement conducteurs est établie par des contacts entre des parties (15) desdits premier et deuxième moyens électriquement conducteurs.

3. Dispositif selon les revendications 1 ou 2, dans lequel les premier et deuxième moyens électriquement conducteurs comprennent une matière choisie dans le groupe constitué de polymères, peintures ou encres électriquement conducteurs.

**4.** Dispositif selon les revendications 1 à 3, comprenant en outre davantage de structures en couches comprenant davantage d'au moins un premier et un deuxième moyen électriquement conducteur et davantage d'au moins un moyen électriquement isolant ; des parties desdits davantage premier et deuxième moyens électriquement conducteurs fournissant des contacts les unes avec les autres pour fournir au moins une connexion entre eux.

**5.** Dispositif selon les revendications 1 à 4, dans lequel ledit au moins un moyen électriquement conducteur comprend un élément inductif comprenant une bobine à enroulements séparés pour recevoir un fluide.

**6.** Dispositif selon les revendications 1 à 5, dans lequel ledit fluide est attiré par le substrat.

**7.** Dispositif selon les revendications 1 à 6, dans lequel la structure en couches est revêtue d'une couche de protection contre le fluide.

**8.** Dispositif selon les revendications 1 à 7, dans lequel le circuit résonnant est désactivé par la présence d'un fluide.

**9.** Dispositif selon les revendications 1 à 8, dans lequel le circuit résonnant comprend en outre un fusible.

**10.** Procédé de fabrication d'un dispositif de détection par circuit résonnant, comprenant les étapes consistant à :

(a) prévoir un substrat (10) comportant une surface ;
(b) prévoir une structure en couches comprenant :

(i) au moins un premier moyen électriquement conducteur (11, 12) comprenant une inductance (L) ;
(ii) au moins un deuxième moyen électriquement conducteur (13) ; et
(iii) au moins un moyen électriquement isolant (14) qui sépare au moins une partie dudit premier moyen électriquement conducteur dudit deuxième moyen électriquement conducteur ;

l'un ou l'autre desdits au moins un premier ou deuxième moyen électriquement conducteur étant placé sur ledit substrat ;
les premier et deuxième moyens électriquement conducteurs et le moyen électriquement isolant étant connectés électriquement pour former un circuit résonnant (LC) ;

dans lequel des parties (11, 13) desdits au moins premier et deuxième moyens électriquement conducteurs sont dotées d'électrodes d'au moins une capacité (C), et
(c) amener des parties (15) desdits premier et deuxième moyens électriquement conducteurs à établir au moins une connexion entre les premier et deuxième moyens électriquement conducteurs ;
ledit circuit résonnant réagissant à un fluide.

**11.** Procédé selon la revendication 10, dans lequel ladite au moins une connexion entre les premier et deuxième moyens électriquement conducteurs est établie par mise en contact mutuel de parties (15) desdits premier et deuxième moyens électriquement conducteurs.

**12.** Procédé selon la revendication 10 ou 11, dans lequel les premier et deuxième moyens électriquement conducteurs comprennent une matière choisie dans le groupe constitué de polymères, peintures ou encres électriquement conducteurs.

**13.** Procédé selon les revendications 10 à 12, comprenant en outre les étapes consistant à prévoir davantage de structures en couches comprenant davantage d'au moins un premier et un deuxième moyen électriquement conducteur et davantage d'au moins un moyen électriquement isolant ; mettre en contact mutuel des parties desdits davantage premier et deuxième moyens électriquement conducteurs pour établir au moins une connexion entre eux.

**14.** Procédé selon les revendications 10 à 13, dans lequel la structure en couches est réalisée par impression.

**15.** Procédé selon les revendications 10 à 14, dans lequel l'impression est commandée par contrôle de paramètres des circuits résonnants fabriqués en ligne et par réglage des conditions du processus d'impression pour obtenir des paramètres souhaités pour ceux-ci.

**16.** Appareil de détection d'un fluide, comprenant : un ou plusieurs oscillateurs (10) pour transmettre de l'énergie électromagnétique ; un ou plusieurs circuits résonnants (11) pour recevoir l'énergie électromagnétique provenant desdits oscillateurs ; et un ou plusieurs détecteurs pour détecter des variations d'une ou de plusieurs caractéristiques desdits un ou plusieurs oscillateurs suite à des variations de caractéristiques des circuits résonnants par contact de ceux-ci avec le fluide, le circuit résonnant étant selon les revendications 1 à 9.

**17.** Appareil selon la revendication 16, dans lequel lesdits un ou plusieurs circuits résonnants comprennent des bobines à enroulements séparés pour recevoir le fluide et court-circuiter le circuit.

**18.** Appareil selon la revendication 16 ou 17, dans lequel les enroulements sont constitués d'une matière électriquement conductrice choisie dans le groupe constitué de polymères électriquement conducteurs tels que la polyaniline ; ou de mélanges de polymères électriquement conducteurs comme des mélanges de poly(p-phénylènevinylène), de polyacrylamide, de polyaniline et de polyéthylène.

**19.** Appareil selon les revendications 16 à 18, dans lequel lesdits un ou plusieurs circuits résonnants sont contenus dans, ou fixés sur, un ou plusieurs contenants.

**20.** Appareil selon les revendications 16 à 19, dans lequel lesdits un ou plusieurs circuits résonnants sont incorporés dans des couches hygiéniques.

**21.** Appareil selon les revendications 16 à 20, dans lequel lesdits un ou plusieurs circuits résonnants sont incorporés dans un support comportant un adhésif comme un autocollant.

**22.** Utilisation d'un appareil selon les revendications 16 à 21 pour détecter le niveau d'un fluide dans un ou plusieurs contenants.

**23.** Utilisation selon la revendication 22, dans lequel les contenants comprennent des contenants et des contenants à infusion.

**24.** Utilisation d'un appareil selon les revendications 16 à 21 pour détecter si un contenant contenant un fluide a été vidé du fluide ou si un contenant vide a été rempli.

**25.** Utilisation selon la revendication 24, dans laquelle le contenant comprend un contenant à infusion.

**26.** Utilisation d'un appareil selon les revendications 16 à 21 pour contrôler une fuite de fluide depuis un contenant.

**27.** Utilisation d'un appareil selon les revendications 16 à 21 pour contrôler une fuite depuis un corps d'homme ou d'animal ou une alimentation en fluide vers un corps d'homme ou d'animal.

**28.** Utilisation selon la revendication 27, dans laquelle la fuite de fluide est un fluide provenant d'un homme atteint d'incontinence urinaire et/ou fécale.

**29.** Article comprenant un dispositif de détection selon les revendications 1 à 9, ou un dispositif de détection fabriqué conformément au procédé selon les revendications 10 à 15 pour contenir ou pour recevoir ou pour administrer un fluide.

**30.** Article comprenant un dispositif de détection selon les revendications 1 à 9, ou un dispositif de détection fabriqué conformément au procédé selon les revendications 10 à 15 pour un développement sain et le maintien de la santé.

**31.** Article selon la revendication 30, constitué d'un absorbant ou d'un bandage sous la forme d'un revêtement ou d'un collecteur utilisé pour protéger, recouvrir ou immobiliser une partie blessée ou malade d'un homme ou d'un animal, ou utilisé en chirurgie.

**32.** Article selon la revendication 31, constitué d'un absorbant pour l'urine ou les matières fécales, en particulier une couche hygiénique.

Fig. 1A

Fig. 1B

Fig. 2A  Fig. 2B  Fig. 2C

Fig. 3A  Fig. 3B  Fig. 3C

Fig. 4A  Fig. 4B  Fig. 4C

Fig. 5A

Fig. 5B

Fig. 5C

Fig. 6A

Fig. 6B

Fig. 6C

10   13

10   13

10   13   61

62

Fig. 7A

Fig. 7B

Fig. 7C

Fig. 8A

Fig. 8B

Fig. 8C

L   C

Fig. 9A    Fig. 9B

Fig. 10A

Fig. 10B

Fig. 11

Fig. 12B

Fig. 12A

Fig. 13B

Fig. 13A

120

60

11

Fig. 14

61

75

72

75

75

11

71

73

Fig. 15

80

81

82

Fig. 16B

80

82

81

Fig. 16A

Fig. 17A

Fig. 17B

Fig. 18A

Fig. 18B